# FASCICULE DE BREVET EUROPEEN

(11) **EP 0 738 511 B1**
(45) Date de publication et mention de la délivrance du brevet: **12.09.2001**
(21) Numéro de dépôt: 96400815.5
(22) Date de dépôt: 17.04.1996
(51) Int. Cl.: A61K 7/48, C10M 119/30, C08G 77/14

(54) **Utilisation de cires silicones à fonctions esters pour épaissir des milieux huileux**
Verwendung von Estergruppe enthaltenden Silikonwachsen als Verdickungsmittel in Oel
Use of silicone waxes containing ester functions as thickeners in oily media

(30) Priorité: 18.04.1995 FR 9504582
(43) Date de publication de la demande: 23.10.1996
(73) Titulaire: RHODIA CHIMIE, 92408 Courbevoie Cédex (FR)
(72) Inventeur: Derian, Paul-Joel, Lawrenceville, NJ 08648 (US)
(74) Mandataire: Fabre, Madeleine-France

(56) Documents cités:
- EP-A- 0 310 903
- EP-A- 0 376 820
- EP-A- 0 515 195
- WO-A-93/17660
- FR-A- 1 488 203

## Description

La présente invention vise l'utilisation de cires silicones à fonctions esters d'acides ou d'alcools gras aliphatiques pour l'épaississement de milieux huileux ou un procédé pour épaissir les milieux huileux par addition desdites cires auxdits milieux, ainsi que les milieux huileux comprenant une cire silicone à fonctions esters d'acides ou d'alcools gras aliphatiques comme agent épaississant.

Il a déjà été proposé d'épaissir les milieux huileux, tels que des huiles hydrocarbonées, des huiles minérales, des huiles végétales, des préparations cosmétiques ou pharmaceutiques, à l'aide d'un polyorganosiloxane présentant des fonctions aliphatiques saturées en C₁₈-C₃₆, ainsi qu'éventuellement des fonctions -(CH₂)ₓ-COOR, où R représente un groupe alkyle en C₁-C₄, avec x pouvant aller de 2 à 12, fonctions directement liées aux atomes de silicium de la chaîne polyorganosiloxane (US-A-4,844,826).

De même, des polyorganosiloxanes à fonctions -R-COOR' et éventuellement -R-OH, où R représente un radical aliphatique divalent en C₂-C₁₈ et R' un radical aliphatique en C₈-C₂₀, radicaux directement liés aux atomes de silicium de la chaîne polyorganosiloxane, ont été mis en oeuvre pour la préparation de compositions cosmétiques de rasage, compositions pouvant se présenter sous forme de crèmes, de gels, de gels automoussants, de mousses aérosol, ou de pains (EP-A-0 376 820).

Le document WO 93 17660 décrit lui une composition cosmétique sous forme de poudre libre ou compacte. Afin de contrôler l'homogénéité, la compacticité et le délitage des poudres, il est proposé d'y introduire des mélanges de silicones comprenant des cires silicones. Ces cires silicones peuvent être des polyorganosiloxanes à fonctions -X- R" directement liées aux atomes de silicium, où R" est un groupement alkyle en C₆-C₃₀ et X représente -(CH₂)ₐ-O-CO - ou -(CH₂)_{b}-CO-O- avec a compris entre 0 et 6 et b entre 6 et 30.

La demanderesse a trouvé que des cires silicones présentant des fonctions de formule -(CH₂)ₙ-A-R, formule dans laquelle n est au moins égal à 2, A un groupe -OCO- ou -COO- et R un groupe aliphatique en C₂₁-C₃₀, plus particulièrement celles présentant des fonctions béhénates -(CH₂)₃-O-CO-(CH₂)₂₀-CH₃, directement liées à des atomes de silicium de la chaîne polyorganosiloxane, étaient tout particulièrement intéressantes pour épaissir certains milieux huileux.

Un premier objet de l'invention consiste en l'utilisation, pour épaissir des milieux huileux dont la phase grasse principale est choisie parmi :
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutènes éventuellement hydrogénés
- les huiles silicones volatiles
de cires silicones à fonctions esters d'acides ou d'alcools gras aliphatiques de formule (I) 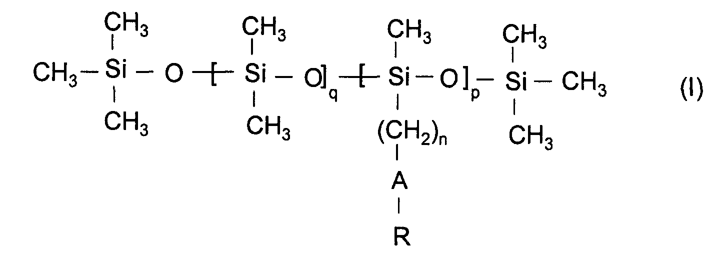 formule dans laquelle :
. n représente un nombre entier au moins égal à 2, de préférence égal à 3
. A représente un groupe 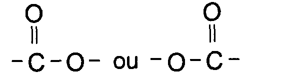
. R représente un groupe aliphatique en C₂₁-C₃₀
. p représente un nombre entier ou décimal pouvant aller de 1 à 100, de préférence de 1 à 10,
. q représente un nombre entier ou décimal pouvant aller de 5 à 100, de préférence de 10 à 100,
la valeur de p / p+q étant telle que le nombre de fonctions -(CH₂)ₙ-A-R présentes dans la cire de formule (I) soit d'environ 50 à 250, de préférence d'environ 100 à 250 milliequivalents pour 100g de ladite cire,
lesdites cires étant choisies parmi celles présentant un point de fusion au moins égal à 40°C.

Les cires silicones de formule (I) peuvent être obtenues par réaction d'hydrosilylation d'un polyhydrogénoorganosiloxane de formule 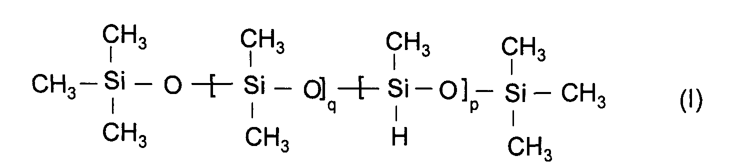 et d'un composé de formule CH₂=CH₂-(CH₂)ₙ₋₂-A-R,
n, p, q, A et R ayant la définition donnée ci-dessus,
en présence d'un catalyseur d'hydrosilylation connu, comme par exemple les complexes du platine.

Parmi les cires silicones de formule (I) préférentielles, on peut citer tout particulièrement les cires silicones à fonctions béhénates, présentant la formule suivante 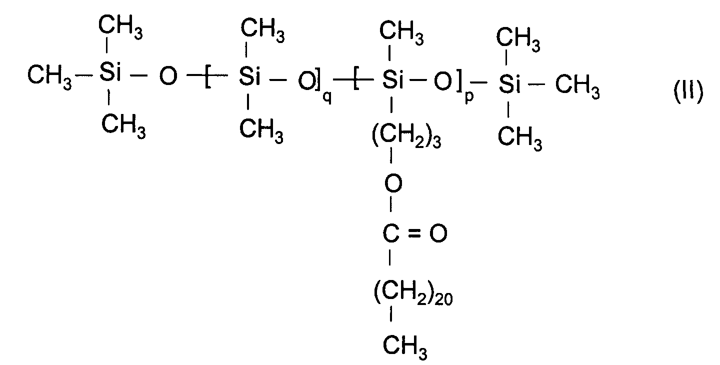 formule dans laquelle p représente un nombre entier ou décimal pouvant aller de 1 à 100, de préférence de 1 à 10, et q représente un nombre entier ou décimal pouvant aller de 5 à 100, de préférence de 10 à 100, la valeur de p / p+q étant telle que le nombre de fonctions béhénates -(CH₂)₃-O-CO-(CH₂)₂₀-CH₃ présentes dans la cire de formule (II) soit d'environ 50 à 250, de préférence d'environ 100 à 250 milliequivalents pour 100g de ladite cire.

Les cires à fonctions béhénates de formule (II) présentent un point de fusion supérieur à 40°C, généralement de l'ordre de 45 à 80°C.

La quantité de cire silicone de formule (I) ou (II) pouvant être utilisée pour épaissir lesdits milieux huileux peut être de l'ordre de 2 à 20, de préférence de l'ordre de 3 à 10 parties en poids pour 100 parties en poids desdits milieux huileux.

Une phase grasse sera considérée comme principale dans un milieu huileux, lorsque celle-ci représente de l'ordre d'au moins 10%, de préférence d'au moins 40% en poids dudit milieu huileux.

On entend par huiles silicones volatiles, les polydiméthylsiloxanes cycliques ou linéaires contenant de 3 à 9, de préférence de 4 à 5 atomes de silicium ; celles-ci présentent une viscosité inférieure à environ 20 mPa.s à 25°C.

Parmi les phases grasses, constituantes des milieux huileux pouvant être ainsi épaissis, on peut citer notamment
- l'alcool oleïque
- l'adipate d'isopropyle
- le polypropylèneglycol (PPG)-3 myristyl éther (WITCONOL APM® commercialisé par WITCO), le PPG-4 myristyl éther, le PPG-4 lauryl éther, le PPG-10 cétyl éther
- les mélanges de cétyl acétate et de lanoline alcool acétylé (CRODALAN LA® commercialisé par CRODA)
- l'acide caprylique triglycéride
- l'huile de maïs
- l'huile de pépins de raisins
- l'huile d'amande douce
- l'huile de jojoba
- le squalane
- les polyisobutènes éventuellement hydrogénés
- l'huile de vaseline
- les huiles paraffiniques telles que l'huile de vaseline
- les huiles isoparaffiniques (MARCHOL 52® et MARCHOL 82® commercialisées par ESSO)
- les huiles silicones volatiles telles que hexaméthylsiloxane, cyclométhicone D4, cyclométhicone D5, seules ou en mélange entre elles ; jusqu'à 20% en poids de ces huiles volatiles peuvent être remplacées par une ou plusieurs huiles silicones ou gommes silicones de viscosité supérieure.
Des milieux huileux composés de mélanges d'émollients cosmétiques comme ceux figurant notamment dans "Cosmetics & Toiletries" / n°93, page 107-juillet 1992, sont particulièrement intéressants.

Ledit milieu huileux à épaissir peut être présent seul ou peut constituer la phase huile d'une émulsion "eau dans huile" ou "huile dans eau" ; il peut être également présent tel quel ou sous forme d'une émulsion simple ou multiple "eau dans huile" ou "huile dans eau", au sein d'une composition cosmétique ou pharmaceutique (crème, lait ...).

La présente invention a également pour objet un procédé pour épaissir des milieux huileux dont la phase grasse principale est choisie parmi
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutènes éventuellement hydrogénés
- les huiles silicones volatiles
par addition auxdits milieux d'au moins une cire silicone à fonctions esters d'acides ou d'alcools gras aliphatiques de formule (I) présentant un point de fusion d'au moins 40°C, de préférence à fonctions béhénates de formule (II) ci-dessus, selon une quantité de l'ordre de 2 à 20, de préférence de l'ordre de 3 à 10 parties en poids pour 100 parties en poids de milieu huileux à épaissir.

Les milieux huileux préférentiels pouvant être mis en oeuvre sont ceux déjà mentionnés ci-dessus.

Ledit procédé peut être favorablement réalisé par mélange du milieu huileux à épaissir et de la cire à fonctions esters d'acides ou d'alcools gras aliphatiques, de préférence béhénates, à une température supérieure à celle du point de fusion de la cire ; ledit mélange peut être réalisé par tout moyen permettant de mélanger deux fluides (pale cadre, retournement ...). On laisse refroidir le mélange sans agitation ; on obtient ainsi un gel ou une phase épaisse.

Un autre objet de l'invention consiste en une composition constituée d'un milieu huileux dont la phase grasse principale est choisie parmi
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutène éventuellement hydrogéné
- les huiles silicones volatiles
ledit milieu étant épaissi à l'aide de l'ordre de 2 à 20, de préférence de l'ordre de 3 à 10% de son poids d'au moins une cire silicone de formule (I) présentant un point de fusion d'au moins 40°C, ou de préférence de formule (II).

Les milieux huileux préférentiels pouvant être présents sont ceux déjà mentionnés ci-dessus.

Ladite composition peut être constituée par ledit milieu huileux épaissi seul ou par une émulsion simple ou multiple "eau dans huile" ou "huile dans eau", émulsion dans laquelle la phase huile est constituée par ledit milieu huileux épaissi ; la dite composition peut également être une formulation cosmétique ou pharmaceutique renfermant ledit milieu épaissi présent tel quel ou sous forme d'une émulsion simple ou multiple "eau dans huile" ou "huile dans eau", à côté d'autres additifs classiques des formulations cosmétiques ou pharmaceutiques.

Les cires silicones à fonctions esters ou béhénates de formule (I) ou (II) sont tout particulièrement intéressantes pour épaissir les milieux huileux entrant dans la composition de gels solaires ou déodorants, notamment pour épaissir les milieux huileux contenant plus de 10%, de préférence plus de 50% de leur poids d'huiles silicones volatiles afin de former des gels translucides.

Les compositions déodorantes ou combattant la transpiration excessive ("anti-perspirant") sont décrites dans la littérature. De telles compositions contiennent généralement des silicones en raison des propriétés sensorielles apportées par ces derniers. Parmi ces propriétés on peut citer par exemple le caratère volatil des silicones cycliques ; les conséquences de cette volatilité sont l'absence de sensation de froid après dépôt sur la peau, un toucher non gras, non collant et un excellent comportement lubrifiant. Ces propriétés hautement appréciables pour donner un caractère cosmétiquement acceptable aux compositions sont transférées aux autres constituants de ces compositions comme les constituants actifs associés à ces silicones. Généralement, les silicones utilisés dans ces compositions sont des polydimethylsiloxanes linéaires ou cycliques (décrits dans le dictionnaire C.T.F.A. - Cosmetic, Toiletries, Fragrance Association -, 5th Edition, 1993) comme des dimethicones, dimethiconols ou des cyclométhicones. Peuvent également être présents d'autres types de silicones seuls ou en association avec ceux précédemment cités comme ceux connus sous les dénominations dimethicone copolyol, diphenyl dimethicone, phenyl dimethicone, amodimethicone ...(dictionnaire CTFA).

On trouve dans les compositions déodorantes ou "antiperspirantes" de nombreux autres composants, soit actifs contre la transpiration ou la formation d'odeurs désagréables, soit modifiant l'aspect physique des compositions, suivant que la composition finale se présente sous la forme d'un liquide, d'un gel ou d'un milieu solidifié. En fonction de leur aspect physique et en fonction du degré de gélification choisi, ces compositions peuvent se présenter sous la forme de bâtons (sticks), gels extrudés, applicateurs à bille, crèmes ...
L'application sur la peau du gel, de la crème épaisse ou gélifiée ou du milieu solidifié est rendue plus agréable lorsque la température de fusion ou de ramollissement du gel est proche de la température de la peau, ce qui se traduit par un effet de glissement lors de l'application.
Les agents épaississants, gélifiants ou solidifiants courants sont les sels de lithium, sodium, potassium, aluminium, zirconium, cerium ..., les acides stéariques, hydroxystéariques, béhéniques, montaniques, les esters d'acides carboxyliques ou hydroxycarboxyliques en C₁₄-C₃₀ de glycol, polyglycol, glycérol, polyglycérol, d'alcools aliphatiques en C₂-C₃₀, les éthers en C₁₄-C₃₀ de polyethylene glycol ou polypropyleneglycol, les alcools aliphatiques en C₁₄-C₃₀ ...
On trouve aussi parmi les agents de texture, les cires conventionnelles ayant des points de fusion entre 30 °C et 150 °C comme la cire d'abeille, le spermaceti, la cire de camauba, la paraffine, les cires microcristallines, la cérésine, l'ozokérite.
Mais toutes ces solutions traditionnelles ne sont généralement pas satisfaisantes pour épaissir les compositions déodorantes ou "antiperspirantes" contenant comme composé volatil principal dans la phase continue le tétradimethylcyclosiloxane ou le pentadiméthylcyclosiloxane, à cause de l'incompatibilité généralement constatée de ces composés avec les silicones qu'ils soient volatils ou non. Cette incompatibilité se traduit généralement, lors de la préparation à chaud de la composition à des températures supérieures à la température de fusion des cires ou des milieux cireux épaississants, par une impossibilité de garantir un milieu homogène dans la composition sans le maintien d'une agitation forte. Cette incompatibilité peut même parfois conduire à une totale séparation de phase et une démixion. Après retour à froid, cette incompatibilité à chaud se traduit par la perte des propriétés épaississantes ou la formation de précipités ou d'agglomérats, néfastes à l'aspect ou au toucher cosmétique de la composition (inhomogénéité, points durs, microcristaux, ...).

Les cires silicones de formules (I) et (II) peuvent être utilisées seules ou en association avec les composés cireux ou les cires précédemment citées comme épaississants. Dans le cas de l'utilisation en association avec d'autres épaississants ou gélifiants, les cires silicones de formules (I) et (II) jouent, outre leur rôle d'épaississant, aussi le rôle de compatibilisant entre les gélifiants et épaississants traditionnels et la phase grasse riche en silicone. Ces cires silicones présentent aussi l'avantage d'éviter aux cires ou milieux cireux traditionnels de former des microcristaux trop gros, qui apporteraient à la composition un toucher désagréable. Les cires silicones jouent ici un rôle d'inhibiteur de la croissance cristalline en co-cristallisant avec les autres cires et en formant ainsi un réseau de microcristallites plus fins qu'en absence de cire silicone. En outre ces cires silicones, utilisées seules ou en association avec les autres cires épaississantes, améliorent le toucher cosmétique de la composition en diminuant le toucher "gras" et en favorisant l'étalement sur la peau.
On trouve parmi les agents actifs utilisés dans les compositions déodorantes et/ou antiperspirantes, des agents astringents limitant la transpiration, des agents antibactériens, des composés absorbants, des additifs contrôlant les odeurs désagéables comme le bis(pyridine)-2-2' disulfide décrit dans EP-A-483428 ou bien les aminoacides décrits dans WO 9111998 ou encore l'acide undécylénique.

Parmi les composés astringents limitant la transpiration et qui peuvent être utilisés dans les compositions déodorantes ou anti-transpiration, on trouve des sels organiques ou inorganiques d'aluminium, de zirconium, de zinc ou leurs sels mixtes ou leurs mélanges.
Ces composés sont décrits ou cités dans la littérature, en particulier dans la revue Cosmetics&Toiletries, Avril 1990, pages 35 à 39. Des exemples de ces composés antiperspirants sont le chlorure d'aluminium, le chlorhydrate d'aluminium et/ou de zirconium, le dichlorhydrate d'aluminium et/ou de zirconium, le trichlorhydrate d'aluminium et/ou de zirconium, le tétratrichlorhydrate d'aluminium et/ou de zirconium, le pentachlorhydrate d'aluminium et/ou de zirconium, le sesquichlorhydrate d'aluminium et/ou de zirconium, l'aluminium chlorhydrex, l'aluminium-zirconium chlorhydrex glycine, l'octachlorhydrate d'aluminium et de zirconium, le sulfate d'aluminium, le sulfate de zinc, le chlorohydroglycinate de zirconium et d'aluminium, l'hydroxychlorure de zirconium, le lactate de zirconium et d'aluminium, le sulfate d'aluminium et de potassium, le chlorohydroxylactate d'aluminium et de sodium, le bromohydrate d'aluminium, le sulfocarbonate de zinc, le bromure d'aluminium, le phenolsulfonate de zinc associé au sulfate d'alminium.
Tous ces composés astringents penvent être en outre encapsulés ou protégés par pelliculage, adsorption, complexation avec des polymères ou par toute autre technique appropriée comme celles décrites dans US-A-4624062.
Des composés bactéricides ou bactériostatiques peuvent être en outre ajoutés à ces compositions déodorantes afin de contrôler la prolifération de la flore microbienne se développant sur le corps. Parmi ces composés on peut citer la chlorhexidine et ses dérivés, la nisine, le trichlosan.
La rémanence de ces composés sur la peau peut être améliorée, si désiré, par addition de 0 à 10 % en poids de polymères comme les polydiméthylsiloxanes et polydimethyldiphénylsiloxanes de haute viscosité, préférentiellement supérieure à 100000 mPa.s.
Les composés astringents sont généralement présents à des concentrations de 1% à 70% en poids, préférentiellement, de 5 à 50% en poids.
Les composés contrôlant la prolifération de la flore bactérienne sur la peau peuvent être introduits à des concentrations de 0,1 à 10 %.
Outre tous ces composés, on peut trouver également dans les compositions déodorantes d'autres composés volatils comme l'éthanol, l'isopropanol, des émollients comme ceux décrits dans la revue Cosmetics&Toiletries, Juillet 1992, N° 107, pages 93 et suivantes, ou les composés comme les alkylmonoglycérides, les alkyldiglycérides, les diols comme le 1-2-propane diol, le 1-3, butane diol, les polyéthylénes glycols ou polypropylénes glycols et leurs esters en C₂-C₁₂, les esters gras liquides comme le palmitate d'isopropyle, le cocoate d'ethyl-2-hexyl, le myristyl myristate, les esters en C₂-C₁₀ d'acides isostéarique ou d'acides hydroxycarboxyliques en C₂-C₈, des silicones non volatils comme les polyalkylsiloxanes linéaires, les silicones copolyols ou copolyethers ..., des parfums, des agents humectants comme le glycérol, le sorbitol, l'urée, le collagène, l'aloe vera, l'acide hyaluronique, les dérivés alcoxylés de sucre ou leurs esters comme les GLUCAM P20® ou le GLUCAM E20® commercialisés par AMERCHOL, de l'eau. Lorsque la composition contient des agents à caractère hydrophile marqué, des agents compatibilisants comme des tensioactifs anioniques, non ioniques, cationiques ou zwitterioniques peuvent être utilisés. Parmi ces tensioactifs, ceux comportant un squelette polyorganosiloxane sont les préférés.

L'utilisation de silicones dans les compositions cosmétiques de protection solaire est connue de longue date.
Notamment les silicones volatils cycliques comme le tetra dimethyl cyclosiloxane ou le penta dimethyl cyclosiloxane sont utilisés pour leur volatilité qui permet un sèchage rapide de la composition appliquée sur la peau sans se traduire par une sensation de froid. En outre, ces silicones volatils apportent aux compositions les propriétés cosmétiques recherchées en facilitant l'étalement des compositions sur la peau et en diminuant le caractère huileux ou gras de ces compositions particulièrement lorsqu'elles sont formulées sous la forme d'huile, de gel solaire ou d'émulsion à phase continue huileuse.
D'une façon générale les silicones sont aussi utilisés pour leurs bonnes propriétés émollientes ou protectrices de la peau. Ces propriétés sont décrites par exemple dans une monographie de l'US Food and Drug Administration (Department of Health and Human Services, Food and Drug Administration, Skin Protectant Drug Products for Over the Counter Human Use, 21 CFR Part 347).
Les silicones les mieux adaptés pour leurs propriétés émollientes sont les polydimethylsiloxanes, les silicones copolyols, les diphenyldimethicones, les phényltrimethicones, les alkylsiloxanes, les dimethiconols, de viscosités comprises entre 20 et 10000 mPa.s.
La résistance à l'eau est essentielle pour assurer la persistance dans le temps de la protection solaire ou anti-UV sur la peau.

L'utilisation de polydimethylsiloxanes de haute masse moléculaire pour assurer une meilleure persistance sur la peau des actifs anti UV est enseignée dans EP-A-197485.
En particulier les polydiméthylsiloxanes de degré de polymérisation supérieure à 5000 unités dimethylsiloxy sont sensiblement résistants au lavage.
Outre ces additifs, les compositions cosmétiques protectrices contre les effets du rayonnement UV contiennent des molécules organiques agissant comme filtres UV, ou des particules minérales agissant comme barrière physique au rayonnement UV. Ces filtres ou adsorbants UV sont bien connus de la littérature ; ils sont par exemple décrits dans l'article de la revue Cosmetics&Toiletries, Vol 102, mars 1987, p21 et suivantes.
On peut citer par exemple les filtres UV comme les para amino benzoates et leurs dérivés, les salicylates, les cinnamates, les benzophénones, le benzylidène camphre, les benzotriazoles et leurs dérivés et d'une manière générale les filtres cités dans l'annexe 7 de la directive européenne 76/768/EEC.
Ces filtres anti UV peuvent être en outre greffés sur une chaîne polymérique en particulier sur une chaine polysiloxanique.
Parmi les particules minérales, on peut citer les particules d'oxyde de titane, d'oxyde de zinc ou de cérium. Ces particules ou nanoparticules d'oxyde minéraux sont éventuellement recouvertes en surface de polymères, de molécules organiques ou d'autres composés minéraux afin d'améliorer leur compatibilité avec les phases organiques et diminuer leur réactivité de surface comme la photocatalyse.
Les cires silicones de formules (I) et (II) peuvent être introduites dans les compositions de protection solaire à des concentrations comprises entre 0,5% et 30% en poids de la composition, préférentiellement de 1% à 20% en poids de cette composition, afin d'épaissir ou gélifier la phase organique hydrophobe de la composition, d'améliorer le toucher de ces compositions en limitant le toucher gras et en facilitant leur étalement, d'augmenter la persistance des agents actifs anti UV sur la peau et afin de maintenir dans le temps l'activité protectrice de la composition déposée sur la peau.
Les cires silicones de formules (I) et (II) peuvent être combinées avec les composés gélifiants ou épaississants comme les cires ou les milieux cireux décrits précédemment.
Outre ces composés, les compositions de protection solaire peuvent comporter des émollients classiquement utilisés en cosmétique, des parfums, des colorants, des pigments ou des laques, des conservateurs, des composés actifs contre l'oxydation ou les radicaux libres, des agents hydratants de la peau et de l'eau.
Les compositions de protection solaire peuvent comporter les composés suivants :
- Cyclomethicone 0-80%
- Autres silicones 0-10%
- Alcools ou éther-alcools volatils 0-50%
   (ou autres composés volatils)
- Emollients, esters 0-30%
- Cire silicone de formule (I) ou (II) 0,5-15%
- Autres cires ou systèmes épaississants 0-10%
- Filtre anti UV 0-15%
- Pigments minéraux 0-10%
- Eau 0-40%
- Parfum, conservateur qsp
Elles peuvent être préparées par mélange des constituants sous agitation à une température supérieure aux points de fusion de la cire silicone et des milieux cireux de la composition, puis coulage de la composition dans un récipient adapté.

Les exemples suivants sont donnés à titre illustratif.

### Exemple 1

On incorpore 10g de la cire silicone à fonctions béhénates (B) définie ci-après
- cire de formule (II) dans laquelle p = 2,5 et q = 13, contenant 80 meq de fonctions béhéniques /100g de polymère et présentant un point de fusion de 48,3°C - dans 90g d'un milieu huileux donné au tableau 1, par mélange à l'aide d'une pale cadre, à une température de 60°C.
30 g du mélange obtenu sont coulés dans un cristallisoir de 5 cm de diamètre.
On laisse refoidir le mélange pendant 24 heures à 20°C.
On constate qu'après refroidissement le gel obtenu ne s'écoule pas lorsqu'on retourne le critallisoir.
Des essais comparatifs ont été réalisés en remplaçant la cire (B) par la cire (B') silicone béhénique ABIL WAX 2440® commercialisée par GOLDSCHMITT, présentant un point de fusion de 42°C et de formule 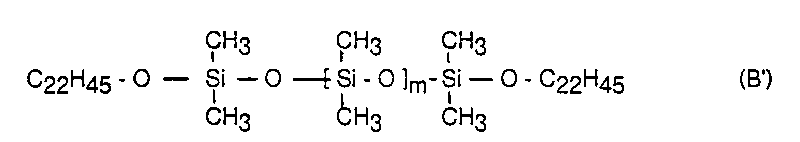 Les résultats obtenus sont les suivants

| **huile** | **B** | **B'** |
|---|---|---|
| alcool oleique | gel | solution |
| adipate d'isopropyle | gel | incompatibilité |
| WITCONOL APM® | gel | solution |
| CRODALAN LA® | gel | solution |
| acide caprylique triglycéride | gel | solution |
| huile de mais | gel | solution |
| huile de pépins de raisin | gel | solution |
| huile d'amande douce | gel | solution |
| huile de jojoba | gel | solution |
| squalane | gel | solution |
| huile de vaseline | gel | solution |
| huile silicone D5 | gel | incompatibilité |

### Exemple 2

Formulation de déodorant :

| | |
|---|---|
| MIRASIL CM4® (cyclométhicone volatile commercialisée par RHONE-POULENC) | 50 % |
| Cire silicone béhénate (B) | 18 % |
| Huile de ricin hydrogénée | 5 % |
| PEG-8 distéarate | 2% |
| Aluminium Zirconium Tetrachlorhydrex-Glyc. (Reach AZP-908) | 20% |
| Talc | 5 % |

Cette formulation est préparée comme suit.
La cire silicone est chauffée jusqu'à 65 °C. On ajoute sous agitation et reflux le silicone volatil, puis on introduit sous agitation les autres composants. L'agitation est maintenue jusqu'à refroidissement à 50 °C, puis la composition est coulée dans des flacons, des sticks ou des moules.

### Exemple 3

Formulation de déodorant

| | |
|---|---|
| MIRASIL CM4® | 35 % |
| MIRASIL CM5-DPDM® (solution de gomme diphényldiméthicone commercialisée par RHONE-POULENC) | 20 % |
| Cire silicone béhenate (B) | 5 % |
| Alcool stéarylique | 10 % |
| Stéarate de sodium | 5% |
| Aluminium Zirconium chlorohydrate | 20 % |
| Propylene glycol | 3% |
| Parfum, colorant | qsp |

Les composés cireux sont chauffés ensemble à 80 °C, puis on ajoute sous agitation et reflux les autres silicones et les autres composants de la formulation. Après homogéneisation, le mélange est refroidi sous agitation jusqu'à 50 °C et coulé dans des récipients adaptés jusqu'à complet refroidissement.

### Exemple 4

Formulation de déodorant

| | |
|---|---|
| MIRASIL CM4® | 55 % |
| Cire silicone béhénate (B) | 10 % |
| Aluminium chlorhydrate | 30 % |
| diméthicone copolyol | 2% |
| Propylene glycol | 2% |
| Parfum | 1 % |

La cire silicone est chauffée à 65 °C. Les autres composés sont ajoutés sous reflux et agitation . Après homogéneisation, le mélange est refroidi sous agitation jusqu'à 50 °C et coulé dans des moules ou récipients adaptés.

### Exemple 5

Formulation d'une émulsion solaire gélifiée

| Phase A : | |
|---|---|
| ARLACEL 1689® (esters de sorbitol et de glycérol commercialisés par ICI) | 3,5% |
| Huile de paraffine | 5,5 % |
| ARLAMOL HD® (heptaméthylnonane commercialisé par ICI) | 8,0 % |
| TIOVEIL TG® (dispersion d'oxyde de titane nanométrique commercialisé par TIOXIDE) | 8,0 % |
| PARSOL MCX® | 2 % |
| Cire silicone béhénate (B) | 5,0 % |

| Phase B : | |
|---|---|
| Glycerol | 4.0 % |
| MgSO₄, 7 H₂O | 0.5% |
| Conservateur | 0,5% |
| eau | qs 100 |

Les phases A et B sont préparées et mélangées à chaud (75 °C) séparément. La phase B est ajoutée lentement sous agitation à la phase A. Après homogénéisation, la composition est laissée refroidir sous agitation jusqu'à 50 °C, puis est transvasée dans un récipient approprié.
La cire silicone béhénate (B) contribue à épaissir la composition et augmente la rémanence des composés anti-UV sur la peau.

## Revendications

1. Utilisation, pour épaissir des milieux huileux dont la phase grasse principale représente de l'ordre d'au moins 10 % en poids desdits milieux huileux et est choisie parmi :
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutènes éventuellement hydrogénés
- les huiles silicones volatiles
de cires silicones à fonctions esters d'acides ou d'alcools gras aliphatiques de formule (I) 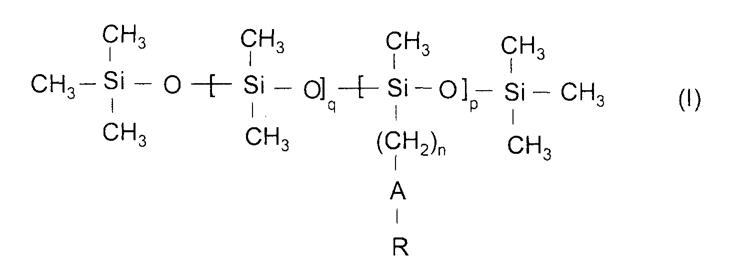 formule dans laquelle :
. n représente un nombre entier au moins égal à 2, de préférence égal à 3
. A représente un groupe 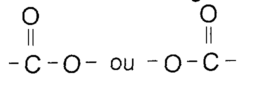
. R représente un groupe aliphatique en C₂₁-C₃₀
. p représente un nombre entier ou décimal pouvant aller de 1 à 100, de préférence de 1 à 10,
. q représente un nombre entier ou décimal pouvant aller de 5 à 100, de préférence de 10 à 100,
la valeur de p / p+q étant telle que le nombre de fonctions -(CH₂)ₙ-A-R présentes dans la cire de formule (I) soit d'environ 50 à 250, de préférence d'environ 100 à 250 milliequivalents pour 100g de ladite cire,
lesdites cires étant choisies parmi celles présentant un point de fusion au moins égal à 40°C.

2. Utilisation selon la revendication 1), **caractérisée en ce que** lesdites cires silicones sont des cires silicones à fonctions béhénates de formule : 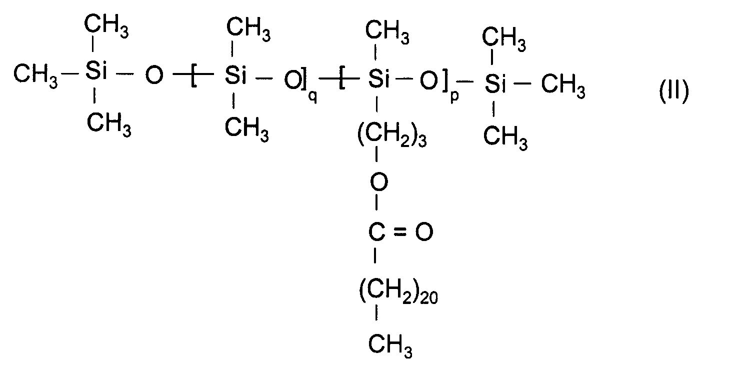 formule dans laquelle p représente un nombre entier ou décimal pouvant aller de 1 à 100, de préférence de 1 à 10, et q représente un nombre entier ou décimal pouvant aller de 5 à 100, de préférence de 10 à 100, la valeur de p / p+q étant telle que le nombre de fonctions béhénates -(CH₂)₃-O-CO-(CH₂)₂₀-CH₃ présentes dans la cire de formule (II) soit d'environ 50 à 250, de préférence d'environ 100 à 250 milliequivalents pour 100g de ladite cire.

3. Utilisation selon la revendication 1) ou 2), **caractérisée en ce que** la quantité de cire silicone de formule (I) ou (II) mise en oeuvre pour épaissir lesdits milieux huileux est de l'ordre de 2 à 20, de préférence de l'ordre de 3 à 10 parties en poids pour 100 parties en poids desdits milieux huileux.

4. Utilisation selon l'une quelconque des revendications précédentes, **caractérisée en ce que** les phases grasses, constituantes des milieux huileux à épaissir, sont
- l'alcool oleïque
- l'adipate d'isopropyle
- le polypropylèneglycol (PPG)-3 myristyl éther, le PPG-4 myristyl éther, le PPG-4 lauryl éther, le PPG-10 cétyl éther
- les mélanges de cétyl acétate et de lanoline alcool acétylé
- l'acide caprylique triglycéride
- l'huile de maïs
- l'huile de pépins de raisins
- l'huile d'amande douce
- l'huile de jojoba
- le squalane
- les polyisobutènes éventuellement hydrogénés
- l'huile de vaseline
- les huiles paraffiniques telles que l'huile de vaseline
- les huiles isoparaffiniques
- les huiles silicones volatiles hexaméthylsiloxane, cyclométhicone D4, cyclométhicone D5, seules ou en mélange entre elles.

5. Utilisation selon l'une quelconque des revendications précédentes, **caractérisée en ce que** ledit milieu huileux à épaissir est présent seul ou peut constituer la phase huile d'une émulsion "eau dans huile" ou "huile dans eau".

6. Utilisation selon l'une quelconque des revendications précédentes, **caractérisée en ce que** ledit milieu huileux à épaissir est présent tel quel ou sous forme d'une émulsion simple ou multiple "eau dans huile" ou "huile dans eau", au sein d'une composition cosmétique ou pharmaceutique.

7. Utilisation selon la revendication 6) **caractérisée en ce que** ladite composition cosmétique est une composition de gel solaire ou de déodorant.

8. Utilisation selon la revendication 7), **caractérisée en ce que** ledit milieu huileux présent au sein de la composition de gel solaire ou de déodorant, contient plus de 10%, de préférence plus de 50% de son poids d'huiles silicones volatiles.

9. Procédé pour épaissir des milieux huileux dont la phase grasse principale représente de l'ordre d'au moins 10 % en poids desdits milieux huileux et est choisie parmi :
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutènes éventuellement hydrogénés
- les huiles silicones volatiles
par addition auxdits milieux d'au moins une cire silicone à fonctions esters d'acides ou d'alcools gras aliphatiques de formule (I) 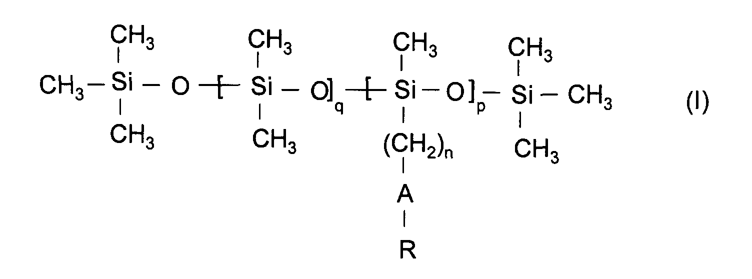 formule dans laquelle :
. n représente un nombre entier au moins égal à 2, de préférence égal à 3
. A représente un groupe 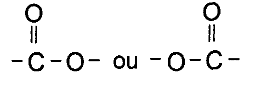
. R représente un groupe aliphatique en C₂₁-C₃₀
. p représente un nombre entier ou décimal pouvant aller de 1 à 100, de préférence de 1 à 10,
. q représente un nombre entier ou décimal pouvant aller de 5 à 100, de préférence de 10 à 100,
la valeur de p / p+q étant telle que le nombre de fonctions -(CH₂)ₙ-A-R présentes dans la cire de formule (I) soit d'environ 50 à 250, de préférence d'environ 100 à 250 milliequivalents pour 100g de ladite cire,
lesdites cires étant choisies parmi celles présentant un point de fusion au moins égal à 40°C.

10. Procédé selon la revendication 9) **caractérisé en ce que** lesdites cires silicones sont des cires silicones à fonctions béhénates de formule : 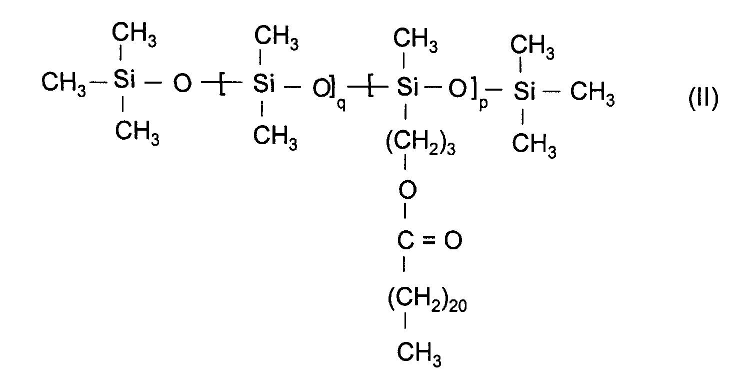 formule dans laquelle p représente un nombre entier ou décimal pouvant aller de 1 à 100, de préférence de 1 à 10, et q représente un nombre entier ou décimal pouvant aller de 5 à 100, de préférence de 10 à 100, la valeur de p / p+q étant telle que le nombre de fonctions béhénates -(CH₂)₃-O-CO-(CH₂)₂₀-CH₃ présentes dans la cire de formule (II) soit d'environ 50 à 250, de préférence d'environ 100 à 250 milliequivalents pour 100g de ladite cire.

11. Procédé selon la revendication 9) ou 10), **caractérisé en ce que** la quantité de cire silicone de formule (I) ou (II) mise en oeuvre pour épaissir lesdits milieux huileux est de l'ordre de 2 à 20, de préférence de l'ordre de 3 à 10 parties en poids pour 100 parties en poids desdits milieux huileux.

12. Procédé selon l'une des revendications 9) à 11), **caractérisé en ce que** les phases grasses, constituantes des milieux huileux à épaissir, sont
- l'alcool oleïque
- l'adipate d'isopropyle
- le polypropylèneglycol (PPG)-3 myristyl éther, le PPG-4 myristyl éther, le PPG-4 lauryl éther, le PPG-10 cétyl éther
- les mélanges de cétyl acétate et de lanoline alcool acétylé
- l'acide caprylique triglycéride
- l'huile de maïs
- l'huile de pépins de raisins
- l'huile d'amande douce
- l'huile de jojoba
- le squalane
- les polyisobutènes éventuellement hydrogénés
- l'huile de vaseline
- les huiles paraffiniques telles que l'huile de vaseline
- les huiles isoparaffiniques
- les huiles silicones volatiles hexaméthylsiloxane, cyclométhicone D4, cyclométhicone D5, seules ou en mélange entre elles.

13. Procédé selon l'une des revendications 9) à 12), **caractérisé en ce que** ledit milieu huileux à épaissir est présent seul ou peut constituer la phase huile d'une émulsion "eau dans huile" ou "huile dans eau".

14. Procédé selon l'une des revendications 9) à 13), **caractérisé en ce que** ledit milieu huileux à épaissir est présent tel quel ou sous forme d'une émulsion simple ou multiple "eau dans huile" ou "huile dans eau", au sein d'une composition cosmétique ou pharmaceutique.

15. Procédé selon la revendication 14), **caractérisé en ce que** ladite composition cosmétique est une composition de gel solaire ou de déodorant.

16. Procédé selon la revendication 15), **caractérisé en ce que** ledit milieu huileux présent au sein de la composition de gel solaire ou de déodorant, contient plus de 10%, de préférence plus de 50% de son poids d'huiles silicones volatiles.

17. Composition constituée d'un milieu huileux dont la phase grasse principale représente de l'ordre d'au moins 10 % en poids du milieu huileux et est choisie parmi
- les alcools gras aliphatiques saturés ou insaturés en C₆-C₂₂, de préférence en C₁₄-C₂₀, contenant éventuellement 1 ou plusieurs hétéroatomes d'oxygène
- les ethers de polyéthylène glycol ou de polypropylène glycol et d'alcools gras en C₄-C₂₂, de préférence en C₁₀-C₂₀
- les esters acétiques d'alcools gras aliphatiques saturés ou insaturés en C₆-C₂₂, de préférence en C₁₄-C₂₀, contenant éventuellement 1 ou plusieurs hétéroatomes d'oxygène
- les diesters aliphatiques de diacides carboxyliques en C₂-C₁₀, de préférence en C₂-C₈
- les triglycérides naturels ou synthétiques
- les huiles isoparaffiniques aliphatiques
- les huiles paraffiniques
- les huiles polyisobutène éventuellement hydrogéné
- les huiles silicones volatiles
ledit milieu étant épaissi à l'aide de l'ordre de 2 à 20, de préférence de l'ordre de 3 à 10% de son poids d'au moins une cire silicone à fonctions béhénates de formule (II) 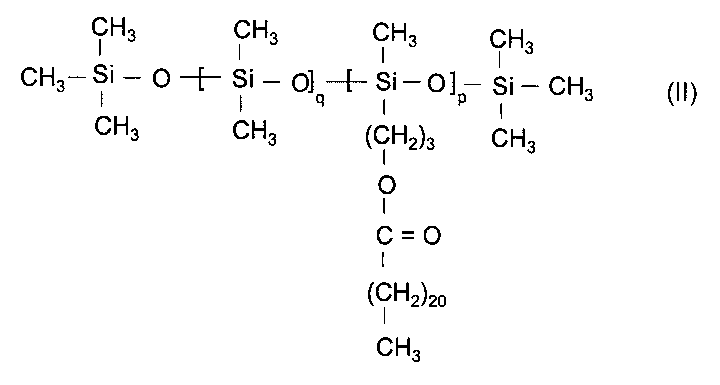 formule dans laquelle p représente un nombre entier ou décimal pouvant aller de 1 à 100, de préférence de 1 à 10, et q représente un nombre entier ou décimal pouvant aller de 5 à 100, de préférence de 10 à 100, la valeur de p / p+q étant telle que le nombre de fonctions béhénates -(CH₂)₃-O-CO-(CH₂)₂₀-CH₃ présentes dans la cire de formule (II) soit d'environ 50 à 250, de préférence d'environ 100 à 250 milliequivalents pour 100 g de ladite cire.

18. Composition selon la revendication 17), **caractérisée en ce que** la quantité de cire silicone de formule (II) est de l'ordre de 2 à 20, de préférence de l'ordre de 3 à 10 parties en poids pour 100 parties en poids desdits milieux huileux.

19. Composition selon l'une quelconque des revendications 17) ou 18), **caractérisée en ce que** les phases grasses, constituantes des milieux huileux, sont
- l'alcool oleïque
- l'adipate d'isopropyle
- le polypropylèneglycol (PPG)-3 myristyl éther, le PPG-4 myristyl éther, le PPG-4 lauryl éther, le PPG-10 cétyl éther
- les mélanges de cétyl acétate et de lanoline alcool acétylé
- l'acide caprylique triglycéride
- l'huile de maïs
- l'huile de pépins de raisins
- l'huile d'amande douce
- l'huile de jojoba
- le squalane
- les polyisobutènes éventuellement hydrogénés
- l'huile de vaseline
- les huiles paraffiniques telles que l'huile de vaseline
- les huiles isoparaffiniques
- les huiles silicones volatiles hexaméthylsiloxane, cyclométhicone D4, cyclométhicone D5, seules ou en mélange entre elles.

20. Composition selon l'une quelconque des revendications 17) à 19), **caractérisée en ce qu'**elle se présente sous forme d'une émulsion "eau dans huile" ou "huile dans eau", emulsion dont la phase huile est constituée par ledit milieu huileux épaissi.

21. Composition selon l'une quelconque des revendications 17) à 20), **caractérisée en ce qu'**elle constitue une composition cosmétique ou pharmaceutique renfermant ledit milieu huileux épaissi, présent tel quel ou sous forme d'une émulsion simple ou multiple "eau dans huile" ou "huile dans eau".

22. Composition selon la revendication 21) **caractérisée en ce que** ladite composition cosmétique est une composition de gel solaire ou de déodorant.

23. Composition selon la revendication 22), **caractérisée en ce que** ledit milieu huileux présent au sein de la composition de gel solaire ou de déodorant, contient plus de 10%, de préférence plus de 50% de son poids d'huiles silicones volatiles.

## Patentansprüche

1. Verwendung von Siliconwachsen mit Gruppen von aliphatischen Fettsäure- oder Fettalkoholestern der Formel (I) 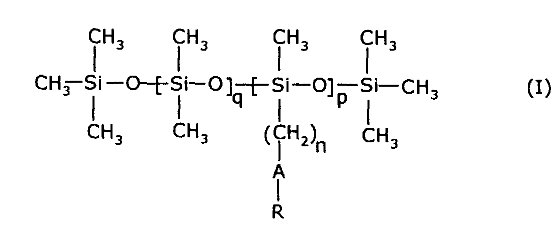 wobei in der Formel:
• n eine ganze Zahl von mindestens 2, vorzugsweise 3, bedeutet
• A eine 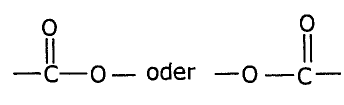 Gruppe bedeutet
• R einen aliphatischen C₂₁-C₃₀-Rest bedeutet
• p eine ganze Zahl oder Dezimalzahl von 1 bis 100, vorzugsweise von 1 bis 10, bedeutet
• q eine ganze Zahl oder Dezimalzahl von 5 bis 100, vorzugsweise von 10 bis 100, bedeutet,
wobei der Wert von p/p+q so ist, daß die Zahl der im Wachs der Formel (I) enthaltenen -(CH₂)ₙ-A-R-Gruppen ungefähr 50 bis 250, vorzugsweise ungefähr 100 bis 250 Milliäquivalente pro 100 g des genannten Wachses ist,
wobei die genannten Wachse aus denen mit einem Schmelzpunkt von mindestens 40 °C gewählt sind,
zum Verdicken von öligen Milieus, deren hauptsächliche Fettphase etwa mindestens 10 Gew.-% der genannten öligen Milieus beträgt und gewählt ist aus:
- den aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den Ethern von Polyethylenglykol oder von Polypropylenglykol und C₄-C₂₂-, vorzugsweise C₁₀-C₂₀-Fettalkoholen,
- den Essigsäureestern der aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den aliphatischen Diestern der C₂-C₁₀-, vorzugsweise C₂-C₈-Dicarbonsäuren,
- den natürlichen oder synthetischen Triglyceriden,
- den aliphatischen Isoparaffinölen,
- den Paraffinölen
- den gegebenenfalls hydrierten Polyisobutenölen
- den flüchtigen Siliconölen.

2. Verwendung nach Anspruch 1, **dadurch gekennzeichnet, daß** die genannten Siliconwachse Siliconwachse mit Behenatgruppen der Formel sind: 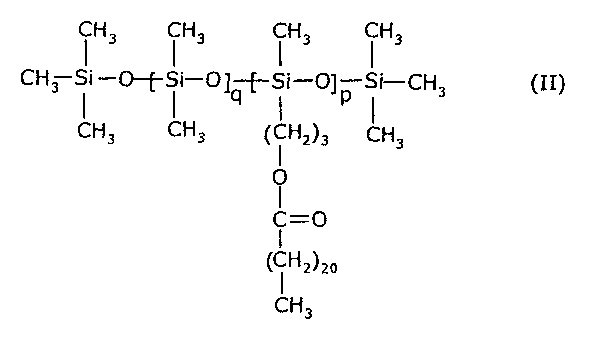 wobei in der genannten Formel p eine ganze Zahl oder Dezimalzahl von 1 bis 100, vorzugsweise von 1 bis 10, und q eine ganze Zahl oder Dezimalzahl von 5 bis 100, vorzugsweise von 10 bis 100, bedeutet, wobei der Wert von p/p+q so ist, daß die Zahl der im Wachs der Formel (II) enthaltenen -(CH₂)₃-O-CO-(CH₂)₂₀-CH₃-Behenatgruppen ungefähr 50 bis 250, vorzugsweise ungefähr 100 bis 250 Milliäquivalente pro 100 g des genannten Wachses ist.

3. Verwendung nach Anspruch 1 oder 2, **dadurch gekennzeichnet, daß** die Menge des zum Verdicken der genannten öligen Milieus verwendeten Wachses der Formel (I) oder (II) etwa 2 bis 20, vorzugsweise etwa 3 bis 10 Gewichtsteile pro 100 Gewichtsteile der genannten öligen Milieus beträgt.

4. Verwendung nach irgendeinem der vorangehenden Ansprüche, **dadurch gekennzeichnet, daß** die Fettphasen, die Bestandteile der zu verdickenden öligen Milieus sind,
- Oleinalkohol
- Isopropyladipat
- Polypropylenglycol (PPG)-3 myristylether, PPG-4 myristylether, PPG-4 laurylether, PPG-10 cetylether
- Gemische von Cetylacetat und acetyliertem Lanolinalkohol
- Caprylsäuretriglycerid
- Maisöl
- Weintraubenkernöl
- Süßmandelöl
- Jojobaöl
- Squalan
- gegebenenfalls hydrierte Polyisobutene
- Vaselinöl
- Paraffinöle wie z.B. Vaselinöl
- Isoparaffinöle
- flüchtige Siliconöle wie z.B. Hexamethylsiloxan, Cyclomethicon D4, Cyclomethicon D5, allein oder als Gemisch miteinander
sind.

5. Verwendung nach irgendeinem der vorangehenden Ansprüche, **dadurch gekennzeichnet, daß** das genannte zu verdickende ölige Milieu allein vorliegt oder die ölige Phase einer "Wasser in Öl"- oder "Öl in Wasser"- Emulsion bilden kann.

6. Verwendung nach irgendeinem der vorangehenden Ansprüche, **dadurch gekennzeichnet, daß** das genannte zu verdickende ölige Milieu unverändert oder in Form einer einfachen oder mehrfachen "Wasser in Öl"- oder "Öl in Wasser"-Emulsion innerhalb einer kosmetischen oder pharmazeutischen Zusammensetzung vorliegt.

7. Verwendung nach Anspruch 6, **dadurch gekennzeichnet, daß** die genannte kosmetische Zusammensetzung eine Sonnenschutzmittel- oder Deodorant-Zusammensetzung ist.

8. Verwendung nach Anspruch 7, **dadurch gekennzeichnet, daß** das innerhalb der Sonnenschutzmittel- oder Deodorant-Zusammensetzung vorliegende ölige Milieu mehr als 10 %, vorzugsweise mehr als 50% seines Gewichtes, an flüchtigen Siliconölen enthält.

9. Verfahren durch Zugabe zu den genannten Milieus mindestens eines Siliconwachses mit Gruppen von aliphatischen Fettäure- oder Fettalkoholestern der Formel (I) 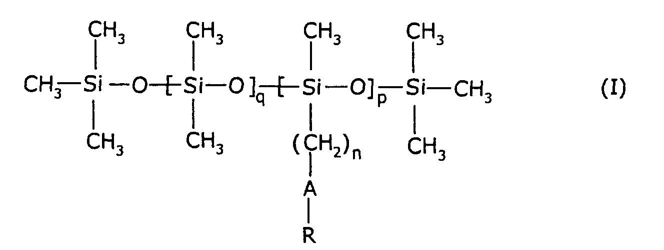 wobei in der Formel:
• n eine ganze Zahl von mindestens 2, vorzugsweise 3, bedeutet
• A eine 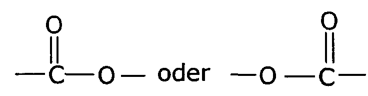 Gruppe bedeutet
• R einen aliphatischen C₂₁-C₃₀-Rest bedeutet
• p eine ganze Zahl oder Dezimalzahl von 1 bis 100, vorzugsweise von 1 bis 10, bedeutet
• q eine ganze Zahl oder Dezimalzahl von 5 bis 100, vorzugsweise von 10 bis 100, bedeutet
wobei der Wert von p/p+q so ist, daß die Zahl der im Wachs der Formel (I) enthaltenen -(CH₂)ₙ-A-R-Gruppen ungefähr 50 bis 250, vorzugsweise ungefähr 100 bis 250 Milliäquivalente pro 100 g des genannten Wachses ist,
wobei die genannten Wachse aus denen mit einem Schmelzpunkt von mindestens 40 °C gewählt sind,
zum Verdicken von öligen Milieus, deren hauptsächliche Fettphase etwa mindestens 10 Gew.-% der genannten öligen Milieus beträgt und gewählt ist aus:
- den aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den Ethern von Polyethylenglykol oder von Polypropylenglykol und C₄-C₂₂-, vorzugsweise C₁₀-C₂₀-Fettalkoholen,
- den Essigsäureestern der aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den aliphatischen Diestern der C₂-C₁₀-, vorzugsweise C₂-C₈-Dicarbonsäuren,
- den natürlichen oder synthetischen Triglyceriden,
- den aliphatischen Isoparaffinölen,
- den Paraffinölen,
- den gegebenenfalls hydrierten Polyisobutenölen,
- den flüchtigen Siliconölen.

10. Verfahren nach Anspruch 9, **dadurch gekennzeichnet, daß** die genannten Siliconwachse Siliconwachse mit Behenatgruppen der Formel sind: 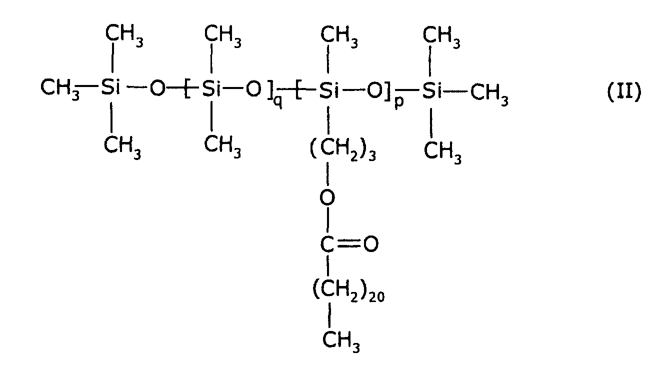 wobei in der genannten Formel p eine ganze Zahl oder Dezimalzahl von 1 bis 100, vorzugsweise von 1 bis 10, und g eine ganze Zahl oder Dezimalzahl von 5 bis 100, vorzugsweise von 10 bis 100, bedeutet, wobei der Wert von p/p+q so ist, daß die Zahl der im Wachs der Formel (II) enthaltenen -(CH₂)₃-O-CO-(CH₂)₂₀-CH₃-Behenatgruppen ungefähr 50 bis 250, vorzugsweise ungefähr 100 bis 250 Milliäquivalente pro 100 g des genannten Wachses ist.

11. Verfahren nach Anspruch 9 oder 10, **dadurch gekennzeichnet, daß** die Menge des zum Verdicken der genannten öligen Milieus verwendeten Wachses der Formel (I) oder (II) etwa 2 bis 20, vorzugsweise etwa 3 bis 10 Gewichtsteile pro 100 Gewichtsteile der genannten öligen Milieus beträgt.

12. Verfahren nach einem der Ansprüche 9 bis 11, **dadurch gekennzeichnet, daß** die Fettenphasen, die Bestandteile der zu verdickenden öligen Milieus sind,
- Oleinalkohol
- Isopropyladipat
- Polypropylenglycol (PPG)-3 myristylether, PPG-4 myristylether, PPG-4 laurylether, PPG-10 cetylether
- Gemische von Cetylacetat und acetyliertem Lanolinalkohol
- Caprylsäuretriglycerid
- Maisöl
- Weintraubenkernöl
- Süßmandelöl
- Jojobaöl
- Squalan
- gegebenenfalls hydrierte Polyisobutene
- Vaselinöl
- Paraffinöle wie z.B. Vaselinöl
- Isoparaffinöle
- flüchtige Siliconöle wie z.B. Hexamethylsiloxan, Cyclomethicon D4, Cyclomethicon D5, allein oder als Gemisch miteinander
sind.

13. Verfahren nach einem der Ansprüche 9 bis 12, **dadurch gekennzeichnet, daß** das genannte zu verdickende ölige Milieu allein vorliegt oder die ölige Phase einer "Wasser in Öl"- oder "Öl in Wasser"-Emulsion bilden kann.

14. Verfahren nach einem der Ansprüche 9 bis 13, **dadurch gekennzeichnet, daß** das genannte zu verdickende ölige Milieu unverändert oder in Form einer einfachen oder mehrfachen "Wasser in Öl"- oder "Öl in Wasser"-Emulsion innerhalb einer kosmetischen oder pharmazeutischen Zusammensetzung vorliegt.

15. Verfahren nach Anspruch 14, **dadurch gekennzeichnet, daß** die genannte kosmetische Zusammensetzung eine Sonnenschutzmittel- oder Deodorant-Zusammensetzung ist.

16. Verfahren nach Anspruch 15, **dadurch gekennzeichnet, daß** das innerhalb der Sonnenschutzmittel- oder Deodorant-Zusammensetzung vorliegende ölige Milieu mehr als 10 %, vorzugsweise mehr als 50 % seines Gewichtes an flüchtigen Siliconölen enthält.

17. Zusammensetzung, die aus einem öligen Milieu besteht, dessen hauptsächliche Fettphase etwa mindestens 10 Gew.-% des öligen Milieus beträgt und gewählt ist aus:
- den aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den Ethern von Polyethylenglykol oder von Polypropylenglykol und C₄-C₂₂-, vorzugsweise C₁₀-C₂₀-Fettalkoholen,
- den Essigsäureestern der aliphatischen, gesättigten oder ungesättigten C₆-C₂₂-, vorzugsweise C₁₄-C₂₀-Fettalkoholen, mit gegebenenfalls einem oder mehreren Sauerstoffheteroatomen,
- den aliphatischen Diestern der C₂-C₁₀-, vorzugsweise C₂-C₈-Dicarbonsäuren,
- den natürlichen oder synthetischen Triglyceriden,
- den aliphatischen Isoparaffinölen,
- den Paraffinölen,
- den gegebenenfalls hydrierten Polyisobutenölen,
- den flüchtigen Siliconölen,
wobei das genannte Milieu mit Hilfe von etwa 2 bis 20, vorzugsweise etwa 3 bis 10 Gew.-% mindestens eines Siliconwachses mit Behenatgruppen verdickt wurde gemäß der Formel (II) 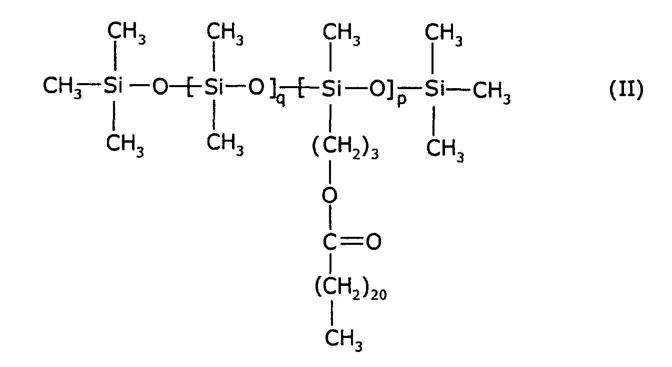 wobei in der genannten Formel p eine ganze Zahl oder Dezimalzahl von 1 bis 100, vorzugsweise von 1 bis 10, und q eine ganze Zahl oder Dezimalzahl von 5 bis 100, vorzugsweise von 10 bis 100, bedeutet, wobei der Wert von p/p+q so ist, daß die Zahl der im Wachs der Formel (II) enthaltenen -(CH₂)₃-O-CO-(CH₂)₂₀-CH₃-Behenatgruppen ungefähr 50 bis 250, vorzugsweise ungefähr 100 bis 250 Milliäquivalente pro 100 g des genannten Wachses ist.

18. Zusammensetzung nach Anspruch 17, **dadurch gekennzeichnet, daß** die Menge des Siliconwachses der Formel (II) etwa 2 bis 20, vorzugsweise etwa 3 bis 10 Gewichtsteile pro 100 Gewichtsteile der genannten öligen Milieus beträgt.

19. Zusammensetzung nach irgendeinem der Ansprüche 17 oder 18, **dadurch gekennzeichnet, daß** die Fettphasen, die Bestandteile der öligen Milieus sind,
- Oleinalkohol
- Isopropyladipat
- Polypropylenglycol (PPG)-3 myristylether, PPG-4 myristylether, PPG-4 laurylether, PPG-10 cetylether
- Gemische von Cetylacetat und acetyliertem Lanolinalkohol
- Caprylsäuretriglycerid
- Maisöl
- Weintraubenkernöl
- Süßmandelöl
- Jojobaöl
- Squalan
- gegebenenfalls hydrierte Polyisobutene
- Vaselinöl
- Paraffinöle wie z.B. Vaselinöl
- Isoparaffinöle
- flüchtige Siliconöle wie z.B. Hexamethylsiloxan, Cyclomethicon D4, Cyclomethicon D5, allein oder als Gemisch miteinander
sind.

20. Zusammensetzung nach irgendeinem der Ansprüche 17 bis 19, **dadurch gekennzeichnet, daß** sie in Form einer "Wasser in Öl"- oder "Öl in Wasser"-Emulsion vorliegt, deren ölige Phase aus dem genannten verdickten öligen Milieu besteht.

21. Zusammensetzung nach irgendeinem der Ansprüche 17 bis 20, **dadurch gekennzeichnet, daß** sie eine kosmetische oder pharmazeutische Zusammensetzung bildet, die das genannte verdickte ölige Milieu enthält, wobei sie unverändert oder in Form einer einfachen oder mehrfachen "Wasser in Öl"- oder "Öl in Wasser"-Emulsion vorliegt.

22. Zusammensetzung nach Anspruch 21, **dadurch gekennzeichnet, daß** die genannte kosmetische Zusammensetzung eine Sonnenschutzmittel- oder Deodorant-Zusammensetzung ist.

23. Zusammensetzung nach Anspruch 22, **dadurch gekennzeichnet, daß** das innerhalb der Sonnenschutzmittel- oder Deodorant-Zusammensetzung vorliegende ölige Milieu mehr als 10 %, vorzugsweise mehr als 50 % seines Gewichtes, an flüchtigen Siliconölen enthält.

## Claims

1. Use, for thickening oily media in which the main fatty phase represents of the order of at least 10% by weight of the said oily media and is chosen from:
- C₆-C₂₂, preferably C₁₄-C₂₀, saturated or unsaturated aliphatic fatty alcohols optionally containing 1 or more oxygen heteroatoms
- ethers of polyethylene glycol or polypropylene glycol and of C₄-C₂₂, preferably C₁₀-C₂₀, fatty alcohols
- acetic esters of C₆-C₂₂, preferably C₁₄-C₂₀, saturated or unsaturated aliphatic fatty alcohols optionally containing 1 or more oxygen heteroatoms
- aliphatic diesters of C₂-C₁₀, preferably C₂-C₈, dicarboxylic acids
- natural or synthetic triglycerides
- aliphatic isoparaffinic oils
- paraffinic oils
- optionally hydrogenated polyisobutene oils
- volatile silicone oils
of silicone waxes containing aliphatic fatty alcohol or acid ester functional groups, of formula (I) 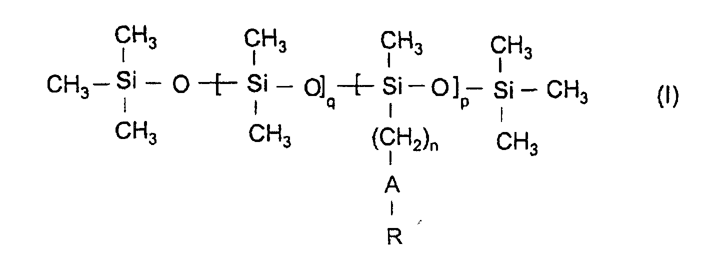 in which formula:
- n denotes an integer equal to at least 2, preferably equal to 3
- A denotes a 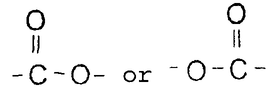 group
- R denotes a C₂₁-C₃₀ aliphatic group
- p denotes a whole or decimal number which can range from 1 to 100, preferably from 1 to 10,
- q denotes a whole or decimal number which can range from 5 to 100, preferably from 10 to 100, the value of p/p+q being such that the number of -(CH₂)ₙ-A-R functional groups present in the wax of formula (I) is from approximately 50 to 250, preferably from approximately 100 to 250, milliequivalents per 100 g of the said wax,
the said waxes being chosen from those which have a melting point equal to at least 40°C.

2. Use according to Claim 1, **characterized in that** the said silicone waxes are silicone waxes containing behenate functional groups, of formula: 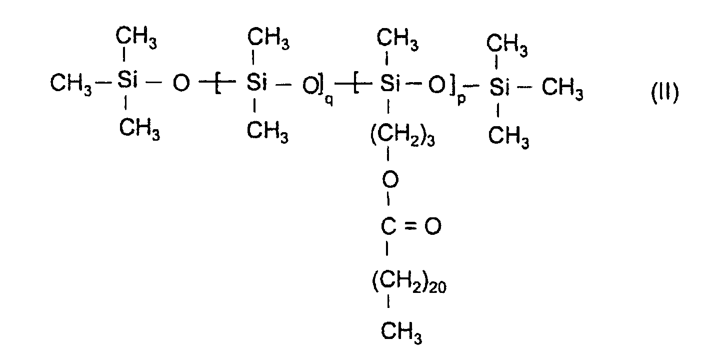 in which formula p denotes a whole or decimal number which can range from 1 to 100, preferably from 1 to 10, and q denotes a whole or decimal number which can range from 5 to 100, preferably from 10 to 100, the value of p/p+q being such that the number of behenate functional groups- (CH₂)₃-O-CO- (CH₂)₂₀-CH₃ present in the wax of formula (II) is from approximately 50 to 250, preferably from approximately 100 to 250, milliequivalents per 100 g of the said wax.

3. Use according to Claim 1 or 2, **characterized in that** the quantity of silicone wax of formula (I) or (II) used for thickening the said oily media is of the order of 2 to 20, preferably of the order of 3 to 10, parts by weight per 100 parts by weight of the said oily media.

4. Use according to any one of the preceding claims, **characterized in that** the fatty phases constituting the oily media to be thickened are
- oleyl alcohol
- isopropyl adipate
- polypropylene glycol (PPG)-3 myristyl ether, PPG-4 myristyl ether, PPG-4 lauryl ether or PPG-10 cetyl ether
- mixtures of cetyl acetate and of acetylated lanolin alcohol
- caprylic acid triglyceride
- corn oil
- grapeseed oil
- sweet almond oil
- jojoba oil
- squalane
- optionally hydrogenated polyisobutenes
- liquid paraffin
- paraffinic oils such as liquid paraffin
- isoparaffinic oils
- volatile silicone oils hexamethylsiloxane, cyclomethicone D4 or cyclomethicone D5, by themselves or mixed with each other.

5. Use according to any one of the preceding claims, **characterized in that** the said oily medium to be thickened is present by itself or may constitute the oil phase of a "water-in-oil" or "oil-in-water" emulsion.

6. Use according to any one of the preceding claims, **characterized in that** the said oily medium to be thickened is present as such or in the form of a simple or multiple "water-in-oil" or "oil-in-water" emulsion within a cosmetic or pharmaceutical composition.

7. Use according to Claim 6, **characterized in that** the said cosmetic composition is an antisun gel or deodorant composition.

8. Use according to Claim 7, **characterized in that** the said oily medium present within the antisun gel or deodorant composition contains more than 10 %, preferably more than 50 %, of its weight of volatile silicone oils.

9. Process for thickening oily media in which the main fatty phase represents of the order of at least 10% by weight of the said oily media and is chosen from:
- C₆-C₂₂, preferably C₁₄-C₂₀, saturated or unsaturated aliphatic fatty alcohols optionally containing 1 or more oxygen heteroatoms
- ethers of polyethylene glycol or polypropylene glycol and of C₄-C₂₂, preferably C₁₀-C₂₀, fatty alcohols
- acetic esters of C₆-C₂₂, preferably C₁₄-C₂₀, saturated or unsaturated aliphatic fatty alcohols optionally containing 1 or more oxygen heteroatoms
- aliphatic diesters of C₂-C₁₀, preferably C₂-C₈, dicarboxylic acids
- natural or synthetic triglycerides
- aliphatic isoparaffinic oils
- paraffinic oils
- optionally hydrogenated polyisobutene oils
- volatile silicone oils
by addition to the said media of at least one silicone wax containing aliphatic fatty alcohol or acid ester functional groups, of formula (I) 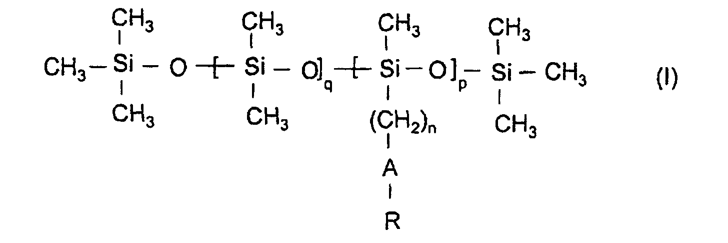 in which formula:
- n denotes an integer equal to at least 2, preferably equal to 3
- A denotes a 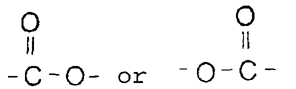 group
- R denotes a C₂₁-C₃₀ aliphatic group
- p denotes a whole or decimal number which can range from 1 to 100, preferably from 1 to 10,
- q denotes a whole or decimal number which can range from 5 to 100, preferably from 10 to 100,
the value of p/p+q being such that the number of -(CH₂)ₙ-A-R functional groups present in the wax of formula (I) is from approximately 50 to 250, preferably from approximately 100 to 250, milliequivalents per 100 g of the said wax, the said waxes being chosen from those which have a melting point equal to at least 40°C.

10. Process according to Claim 9, **characterized in that** the said silicone waxes are silicone waxes containing behenate functional groups, of formula: 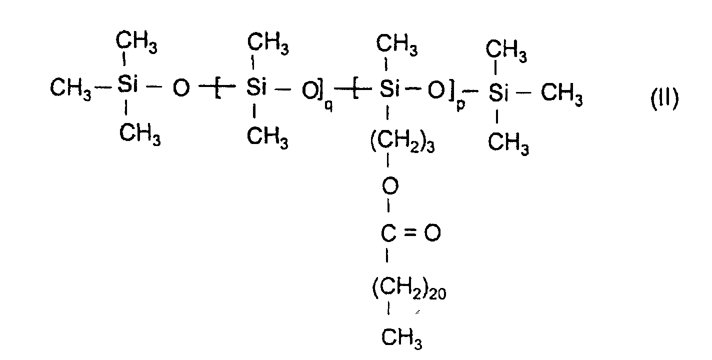 in which formula p denotes a whole or decimal number which can range from 1 to 100, preferably from 1 to 10, and q denotes a whole or decimal number which can range from 5 to 100, preferably from 10 to 100, the value of p/p+q being such that the number of behenate functional groups -(CH₂)₃-O-CO-(CH₂)₂₀-CH₃ present in the wax of formula (II) is from approximately 50 to 250, preferably from approximately 100 to 250, milliequivalents per 100 g of the said wax.

11. Process according to Claim 9 or 10, **characterized in that** the quantity of silicone wax of formula (I) or (II) used to thicken the said oily media is of the order of 2 to 20, preferably of the order of 3 to 10, parts by weight per 100 parts by weight of the said oily media.

12. Process according to one of Claims 9 to 11, **characterized in that** the fatty phases constituting the oily media to be thickened are:
- oleyl alcohol
- isopropyl adipate
- polypropylene glycol (PPG)-3 myristyl ether, PPG-4 myristyl ether, PPG-4 lauryl ether or PPG-10 cetyl ether
- mixtures of cetyl acetate and of acetylated lanolin alcohol
- caprylic acid triglyceride
- corn oil
- grapeseed oil
- sweet almond oil
- jojoba oil
- squalane
- optionally hydrogenated polyisobutenes
- liquid paraffin
- paraffinic oils such as liquid paraffin
- isoparaffinic oils
- volatile silicone oils hexamethylsiloxane, cyclomethicone D4 or cyclomethicone D5, by themselves or mixed with each other.

13. Process according to one of Claims 9 to 12, **characterized in that** the said oily medium to be thickened is present by itself or may constitute the oil phase of a "water-in-oil" or "oil-in-water" emulsion.

14. Process according to one of Claims 9 to 13, **characterized in that** the said oily medium to be thickened is present as such or in the form of a simple or multiple "water-in-oil" or "oil-in-water" emulsion within a cosmetic or pharmaceutical composition.

15. Process according to Claim 14, **characterized in that** the said cosmetic composition is an antisun gel or deodorant composition.

16. Process according to Claim 15, **characterized in that** the said oily medium present within the antisun gel or deodorant composition contains more than 10 %, preferably more than 50 %, of its weight of volatile silicone oils.

17. Composition consisting of an oily medium in which the main fatty phase represents of the order of at least 10% by weight of the oily medium and is chosen from
- C₆-C₂₂, preferably C₁₄-C₂₀, saturated or unsaturated aliphatic fatty alcohols optionally containing 1 or more oxygen heteroatoms
- ethers of polyethylene glycol or polypropylene glycol and of C₄-C₂₂, preferably C₁₀-C₂₀, fatty alcohols
- acetic esters of C₆-C₂₂, preferably C₁₄-C₂₀, saturated or unsaturated aliphatic fatty alcohols optionally containing 1 or more oxygen heteroatoms
- aliphatic diesters of C₂-C₁₀, preferably C₂-C₈, dicarboxylic acids
- natural or synthetic triglycerides
- aliphatic isoparaffinic oils
- paraffinic oils
- optionally hydrogenated polyisobutene oils
- volatile silicone oils
the said medium being thickened with the aid of the order of 2 to 20, preferably of the order of 3 to 10, % of its weight of at least one silicone wax containing behenate functional groups, of formula (II): 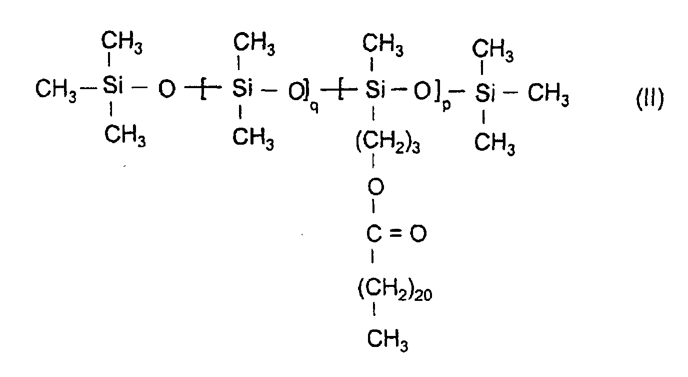 in which formula p denotes a whole or decimal number which can range from 1 to 100, preferably from 1 to 10, and q denotes a whole or decimal number which can range from 5 to 100, preferably from 10 to 100, the value of p/p+q being such that the number of behenate functional groups -(CH₂)₃-O-CO-(CH₂)₂₀-CH₃ present in the wax of formula (II) is from approximately 50 to 250, preferably from approximately 100 to 250, milliequivalents per 100 g of the said wax.

18. Composition according to Claim 17, **characterized in that** the quantity of silicone wax of formula (II) is of the order of 2 to 20, preferably of the order of 3 to 10, parts by weight per 100 parts by weight of the said oily media.

19. Composition according to either of Claims 17 and 18, **characterized in that** the fatty phases constituting the oily media are
- oleyl alcohol
- isopropyl adipate
- polypropylene glycol (PPG)-3 myristyl ether, PPG-4 myristyl ether, PPG-4 lauryl ether or PPG-10 cetyl ether
- mixtures of cetyl acetate and of acetylated lanolin alcohol
- caprylic acid triglyceride
- corn oil
- grapeseed oil
- sweet almond oil
- jojoba oil
- squalane
- optionally hydrogenated polyisobutenes
- liquid paraffin
- paraffinic oils such as liquid paraffin
- isoparaffinic oils
- volatile silicone oils hexamethylsiloxane, cyclomethicone D4 or cyclomethicone D5, by themselves or mixed with each other.

20. Composition according to any one of Claims 17 to 19, **characterized in that** it is in the form of a "water-in-oil" or "oil-in-water" emulsion, in which emulsion the oil phase consists of the said thickened oily medium.

21. Composition according to any one of Claims 17 to 20, **characterized in that** it constitutes a cosmetic or pharmaceutical composition containing the said thickened oily medium, present as such or in the form of a simple or multiple "water-in-oil" or "oil-in-water" emulsion.

22. Composition according to Claim 21, **characterized in that** the said cosmetic composition is an antisun gel or deodorant composition.

23. Composition according to Claim 22, **characterized in that** the said oily medium present within the antisun gel or deodorant composition contains more than 10 %, preferably more than 50 %, of its weight of volatile silicone oils.
